# EUROPEAN PATENT APPLICATION

(11) **EP 4 759 256 A1**
(43) Date of publication of application: **17.06.2026**
(21) Application number: 25222186.6
(22) Date of filing: 10.12.2025
(51) Int. Cl.: A61B 18/08, A61B 18/10, A61B 18/14

(54) **SURGICAL END EFFECTOR WITH BIPOLAR AND ISOLATED MONOPOLAR ELECTRODES**

(30) Priority: 10.12.2024 US 202418975401
(71) Applicant: Cilag GmbH International, 6300 Zug (CH)
(72) Inventor: JAYME, Madeleine C., Cincinnati, 45242 (US); WELLS, Nohemi C., Cincinnati, 45242 (US); WAID, Christopher J., Cincinnati, 45242 (US); VENDELY, Michael J., Cincinnati, 45242 (US)
(74) Representative: Carpmaels & Ransford LLP

(57) **Abstract**

A surgical instrument, including a shaft assembly and an end effector. The end effector including a first jaw, a second jaw, a first bipolar electrode, a second bipolar electrode, and a first monopolar electrode. The first jaw having a first inner portion and a first outer portion. The second jaw having a second inner portion and a second outer portion and being configured to selectively move relative to the first jaw. The first bipolar electrode being secured to the first inner portion of the first jaw or the second inner portion of the second jaw. The second bipolar electrode being secured to the first inner portion of the first jaw or the second inner portion of the second jaw. The first monopolar electrode being secured to the first outer portion of the first jaw or the second outer portion of the second jaw.

## Description

### BACKGROUND

A variety of surgical instruments include a tissue cutting element and one or more elements that transmit radio frequency (RF) energy to tissue (e.g., to coagulate or seal the tissue). An example of such an electrosurgical instrument is the ENSEAL^{®} Tissue Sealing Device by Ethicon Endo-Surgery, Inc., of Cincinnati, Ohio. While bipolar energy is frequently a desired energy modality for these electrosurgical instruments, one or more procedures may benefit from differing energy modalities, even sequentially during a given procedure. Such sequential applications of different energy modalities often includes use of differing instruments, which requires additional costs and time to insert and remove different instruments in the same procedure, thereby decreasing the likelihood of positive outcomes for a patient.

While a variety of surgical instruments have been made and used, it is believed that no one prior to the inventors has made or used the invention described in the appended claims.

### BRIEF DESCRIPTION OF THE DRAWINGS

While the specification concludes with claims which particularly point out and distinctly claim this technology, it is believed this technology will be better understood from the following description of certain examples taken in conjunction with the accompanying drawings, in which like reference numerals identify the same elements and in which:
FIG. 1 depicts a perspective view of an example of a electrosurgical instrument;
FIG. 2 depicts a perspective view of an example of an articulation assembly and a first example end effector of the electrosurgical instrument of FIG. 1;
FIG. 3 depicts a side view of a second example of an end effector for incorporation into an electrosurgical instrument, such as the electrosurgical instrument of FIG. 1, with the end effector in a closed configuration;
FIG. 4 depicts a front view of the end effector of FIG. 3 in the closed configuration;
FIG. 5 depicts a side view of the end effector of FIG. 3 in an open configuration;
FIG. 6A depicts a side view of the end effector of FIG. 3 in the closed configuration wedged into tissue;
FIG. 6B depicts the side view of the end effector similar to FIG. 6A, but with the end effector in the open configuration and spreading the tissue; and
FIG. 6C depicts the side view of the end effector similar to FIG. 6B, but with the end effector in the closed configuration and a first portion of the tissue spread apart from another portion of the tissue.

The drawings are not intended to be limiting in any way, and it is contemplated that various embodiments of the technology may be carried out in a variety of other ways, including those not necessarily depicted in the drawings. The accompanying drawings incorporated in and forming a part of the specification illustrate several aspects of the present technology, and together with the description explain the principles of the technology; it being understood, however, that this technology is not limited to the precise arrangements shown.

### DETAILED DESCRIPTION

The following description of certain examples of the technology should not be used to limit its scope. Other examples, features, aspects, embodiments, and advantages of the technology will become apparent to those skilled in the art from the following description, which is by way of illustration, one of the best modes contemplated for carrying out the technology. As will be realized, the technology described herein is capable of other different and obvious aspects, all without departing from the technology. Accordingly, the drawings and descriptions should be regarded as illustrative in nature and not restrictive.

It is further understood that any one or more of the teachings, expressions, embodiments, examples, etc. described herein may be combined with any one or more of the other teachings, expressions, embodiments, examples, etc. that are described herein. The following-described teachings, expressions, embodiments, examples, etc. should therefore not be viewed in isolation relative to each other. Various suitable ways in which the teachings herein may be combined will be readily apparent to those of ordinary skill in the art in view of the teachings herein. Such modifications and variations are intended to be included within the scope of the claims.

For clarity of disclosure, the terms "proximal" and "distal" are defined herein relative to a surgeon or other operator grasping a surgical instrument having a distal surgical end effector. The term "proximal" refers the position of an element closer to the surgeon or other operator and the term "distal" refers to the position of an element closer to the surgical end effector of the surgical instrument and further away from the surgeon or other operator.

### I. Example of Electrosurgical Instrument with Bipolar and Monopolar Electrodes

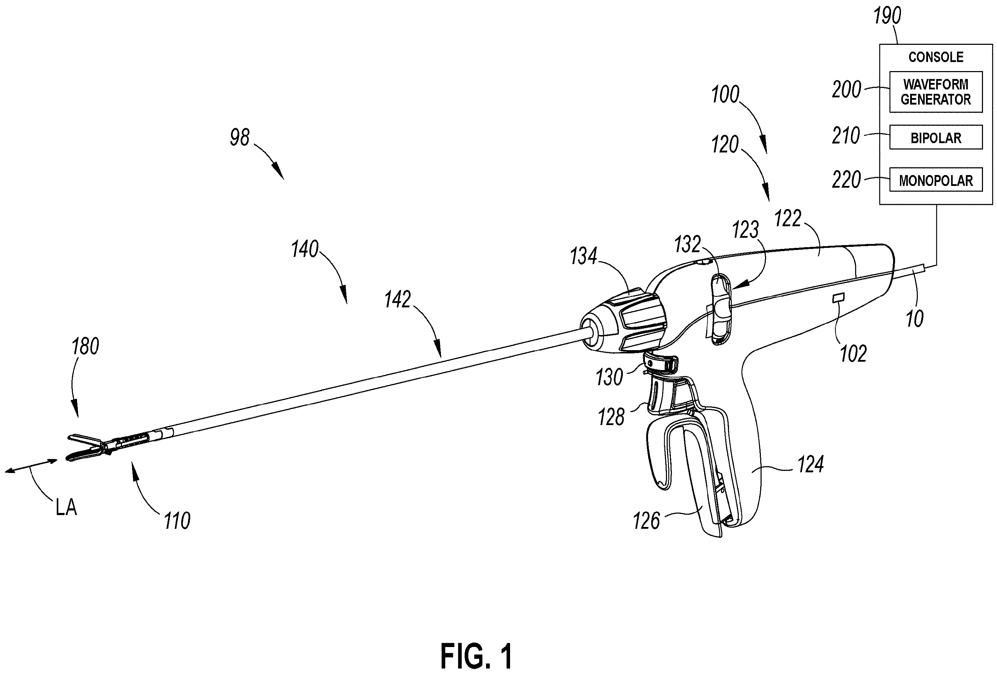

FIGS. 1-2 show a surgical system (98) including an example of electrosurgical instrument (100). As best seen in FIG. 1, electrosurgical instrument (100) includes a handle assembly (120), a shaft assembly (140), an articulation assembly (110), which may also be referred to as an articulation section (110), and an end effector (180). As will be described in greater detail below, end effector (180) of electrosurgical instrument (100) is operable to grasp, cut, and seal or weld tissue (e.g., a blood vessel, etc.). In this example, end effector (180) is configured to apply a non-therapeutic bipolar radio frequency (RF) energy in order to identify and/or verify that the correct tissue is present in the end effector such that a therapeutic RF energy can be applied to seal or weld tissue. However, it should be understood that electrosurgical instrument (100) may be configured to seal or weld tissue through any other suitable means that would be apparent to one skilled in the art in view of the teachings herein. For example, electrosurgical instrument (100) may be configured to seal or weld tissue via an ultrasonic blade, staples, etc. In the present example, electrosurgical instrument (100) is electrically coupled to a waveform generator (200) of surgical system (98), which is capable of delivering therapeutic and non-therapeutic energy, via power cable (10).

Console (190) includes a waveform generator (200), a bipolar power supply (210), and a monopolar power supply (220). Waveform generator (200) may be configured to provide all or some of the electrical power requirements for use of electrosurgical instrument (100). Any suitable waveform generator (200) may be used as would be apparent to one skilled in the art in view of the teachings herein. By way of non-limiting example, the waveform generator (200) may be constructed in accordance with at least some of the teachings of U.S. Pat. No. 8,986,302, entitled "Surgical Generator for Ultrasonic and Electrosurgical Devices," issued March 24, 2015, the disclosure of which is incorporated by reference herein, in its entirety. Bipolar power supply (210) and monopolar power supply (220) may also be incorporated into console (190) to provide power to respective bipolar electrodes and monopolar electrodes, as will be described below. Console (190) may include a selectable input for a user to select whether they want to send power separately or in combination through any of waveform generator (200), bipolar power supply (210), and monopolar power supply (220)

While in the current example, electrosurgical instrument (100) is coupled to console (190) via power cable (10), electrosurgical instrument (100) may contain an internal power source or plurality of power sources, such as a battery and/or supercapacitors, to electrically power electrosurgical instrument (100). Of course, any suitable combination of power sources may be utilized to power electrosurgical instrument (100) as would be apparent to one skilled in the art in view of the teaching herein.

Handle assembly (120) is configured to be grasped by an operator with one hand, such that an operator may control and manipulate electrosurgical instrument (100) with a single hand. Although electrosurgical instrument (100) is primarily described herein as being used by a human user, it should be noted that alternative versions exist in which one or more robotic systems (e.g., a robotic arm) may be used to control and manipulate electrosurgical instrument (100). Shaft assembly (140) extends distally from handle assembly (120) and connects to articulation assembly (110). Articulation assembly (110) is also connected to a proximal end of end effector (180). As will be described in greater detail below, components of handle assembly (120) are configured to control end effector (180) such that an operator may grasp, cut, and seal or weld tissue. Articulation assembly (110) is configured to deflect end effector (180) from the longitudinal axis (LA) defined by shaft assembly (140).

Handle assembly (120) of the present example includes a control unit (102), which may also be referred to herein as a controller (102), housed within a body (122), a pistol grip (124), a jaw closure trigger (126), a knife trigger (128), an activation button (130), an articulation control (132), and a knob (134). As will be described in greater detail below, jaw closure trigger (126) may be pivoted toward and away from pistol grip (124) and/or body (122) to open and close jaws (182, 184) of end effector (180) to grasp tissue. Additionally, knife trigger (128) may be pivoted toward and away from pistol grip (124) and/or body (122) to actuate a knife member (176) within the confines of jaws (182, 184) to cut tissue captured between jaws (182, 184). Further, activation button (130) may be pressed to apply bipolar and/or monopolar radio frequency (RF) energy to tissue via bipolar electrodes (194, 196) and/or monopolar electrodes (170, 172) of jaws (182, 184), respectively. In some versions, bipolar electrodes (194, 196) of jaws (182, 184) are in a bifurcation configuration where bipolar electrodes (194, 196) move relative to a central axis and nearly equal and opposite to one another.

Body (122) of handle assembly (120) defines an opening (123) through which a portion of articulation control (132) protrudes. Articulation control (132) is rotatably disposed within body (122) such that an operator may rotate the portion of articulation control (132) protruding from opening (123) to rotate the portion of articulation control (132) located within body (122). Rotation of articulation control (132) relative to body (122) will bend articulation assembly (110) in order to drive deflection of end effector (180) from the longitudinal axis (LA) defined by shaft assembly (140). Articulation control (132) and articulation assembly (110) may include any suitable features to drive deflection of end effector (180) from the longitudinal axis (LA) defined by shaft assembly (140) as would be apparent to one skilled in the art in view of the teachings herein.

Knob (134) is rotatably disposed on the distal end of body (122) and is configured to rotate end effector (180), articulation assembly (110), and shaft assembly (140) about the longitudinal axis (LA) of shaft assembly (140) relative to handle assembly (120). While in the current example, end effector (180), articulation assembly (110), and shaft assembly (140) are rotated by knob (134), knob (134) may be configured to rotate end effector (180) and articulation assembly (110) relative to selected portions of shaft assembly (140). Knob (134) may include any suitable features to rotate end effector (180), articulation assembly (110), and shaft assembly (140) as would be apparent to one skilled in the art in view of the teachings herein.

Shaft assembly (140) includes distal portion (142) extending distally from handle assembly (120) and a proximal portion housed within the confines of body (122) of handle assembly (120). Shaft assembly (140) houses a portion of knife member (176) extending between distal a distal cutting edge (178) of knife member (176) and knife trigger (128). Shaft assembly (140) also houses actuating members (112) that couple articulation assembly (110) with articulation control (132); as well as an electrical coupling (not shown) that operatively couples electrodes (194, 196) with activation button (130). Knife member (176) is coupled to knife trigger (128) of handle assembly (120) to translate distal cutting edge (178) within the confines of end effector (180); and activation button (130) is configured to activate electrodes (194, 196).

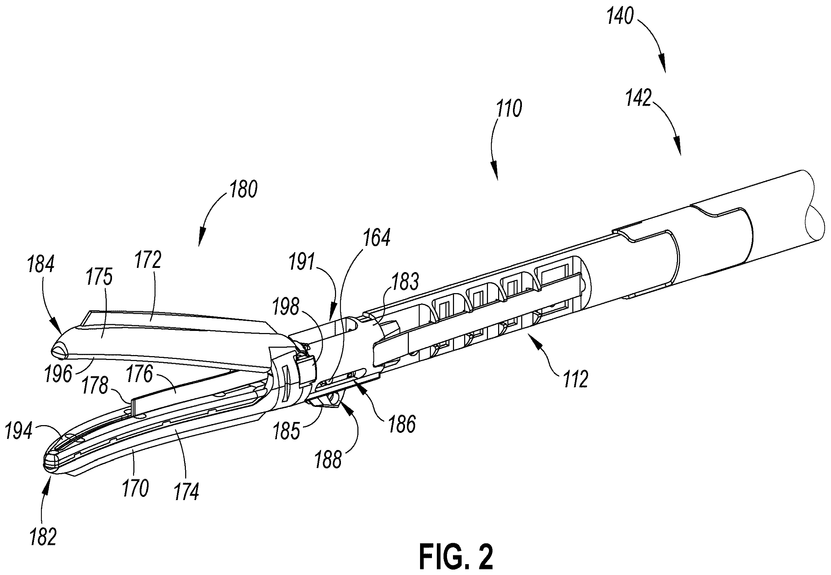

As best seen in FIG. 2, end effector (180) includes lower jaw (182) pivotally coupled with upper jaw (184) via pivot couplings (198). While FIGS. 1-2 show upper jaw (184) being pivotable, this is not to be construed as limiting since there may be alternative examples where both lower jaw (182) and upper jaw (184) pivot relative to articulation assembly (110). In the current example, lower jaw (182) includes a proximal body (183) defining a slot (186), while upper jaw (184) includes proximal arms (185) defining a slot (188). Lower jaw (182) also defines a central channel (191) that is configured to receive proximal arms (185) of upper jaw (184), portions of knife member (176), and pin (164). Slots (186, 188) each slidably receive pin (164), which is attached to a distal coupling portion (162).

With continued reference to FIG. 2, lower jaw (182) is curved and includes monopolar electrode (170). Similarly, upper jaw (184) is also curved and includes a similar monopolar electrode (172). Monopolar electrodes (170, 172) may be similarly curved and thus follow along a whole length or a portion of the length of respective upper and lower jaws (182, 184). As shown, monopolar electrodes (170, 172) are in the form of a fin extending upwardly and downwardly away from respective upper and lower jaws (182, 184). Monopolar electrodes (170, 172) may include a height of approximately 3mm and a lateral width which is smaller than the height. Monopolar electrodes (170, 172) are in electrical communication with monopolar power supply (220) and are capable of delivering monopolar energy to adjacent tissue for cutting and/or cauterization of the tissue. Monopolar power supply (220) may include a selectable input (not shown) capable of selecting a particular monopolar electrodes (170, 172) or both to thereby energize.

Insulating layers (174, 175) of upper and lower jaws (182, 184) electrically isolate monopolar electrodes (170, 172) from respective bipolar electrodes (194, 196). Further bipolar power supply (210) and monopolar power supply (220) are electrically isolated from one another within console (190) and along power cable (10). By electrically isolating the monopolar and bipolar components, a user may ensure that electricity does not inadvertently transfer between them.

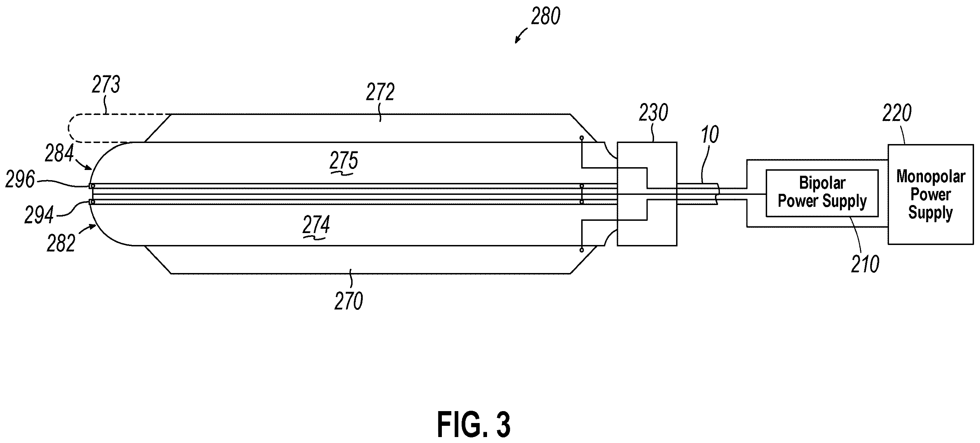

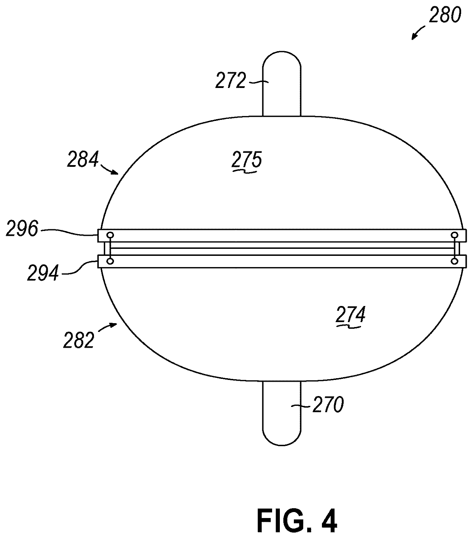

FIGS. 3-5 depict a second example of an end effector (280) which may be substantially similar in form and function to end effector (180) but for the following differences. End effector (280) includes upper jaw (284) and lower jaw (282) which are both straight jaws, linearly extending in a longitudinal direction. Upper jaw (284) includes a monopolar electrode (272), an insulating layer (275), and a bipolar electrode (294) such that insulating layer (275) electrically isolates monopolar electrode (272) from bipolar electrode (294). Lower jaw (282) may include similar components such as monopolar electrode (270), insulating layer (274), and bipolar electrode (294). In an alternative example, monopolar electrode (272) may include a bulbous tip (273) extending from a distal end thereof. Bulbous tip (273) may extend distally beyond remaining portions of end effector (280) in the longitudinal direction to thus be the distal most extending portion of end effector (280). Alternatively, bulbous tip (273) may not extend beyond remaining portions of end effector (280) such that bulbous tip is entirely proximal to another portion of end effector (280). Bulbous tip (273) may include a sharp distal end for cutting tissue or a blunt end for moving tissue.

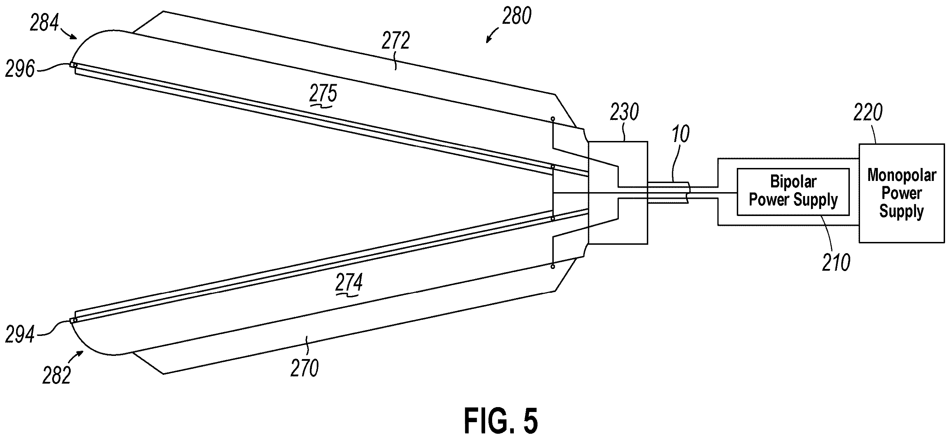

FIG. 5 depicts end effector (280) transitioned from a closed configuration, as shown in FIGS. 3-4, to an open configuration where each of jaws (282, 284) are pivotable relative to articulation section (230). Each jaw (282, 284) may be selectively pivotable relative to articulation section (230) independent of the other jaw (282, 284). One jaw (282, 284) may be fixedly secured to articulation section (230) while the other jaw (282, 284) may be pivotable relative to articulation section (230). Monopolar power supply (220) is capable of energizing monopolar electrodes (270, 272) in both of the open and closed configurations such that energy may be passed to adjacent tissue.

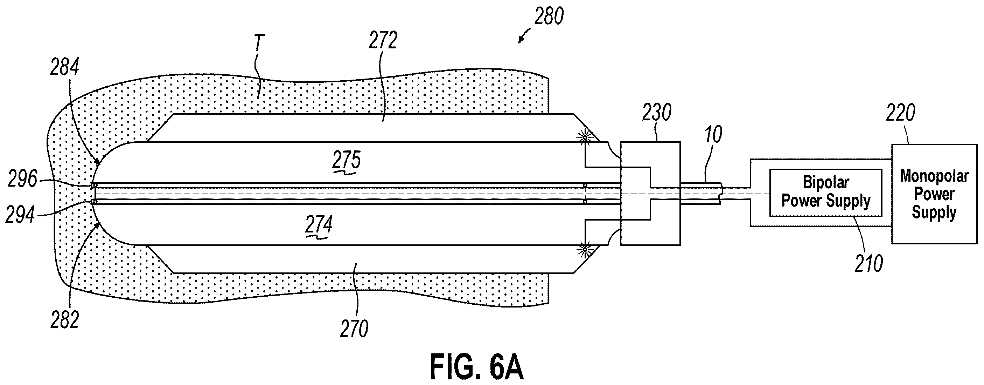

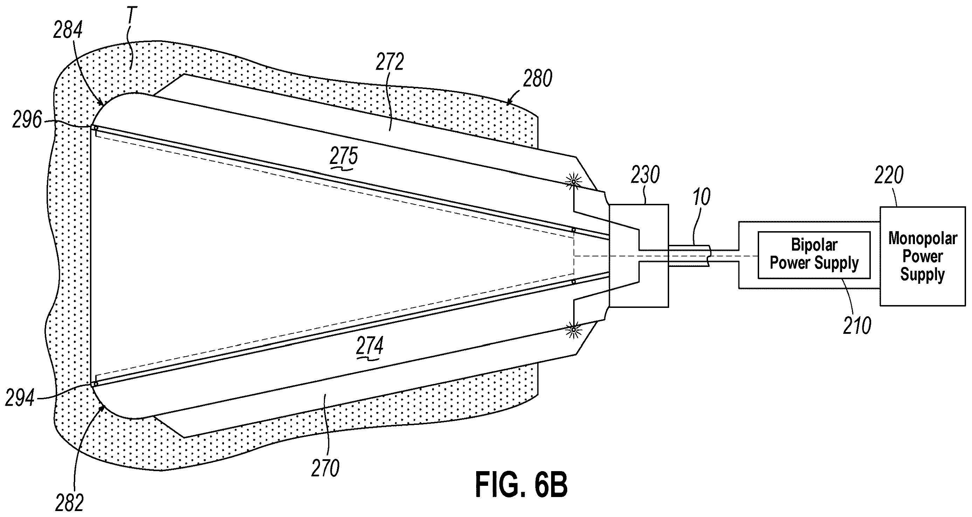

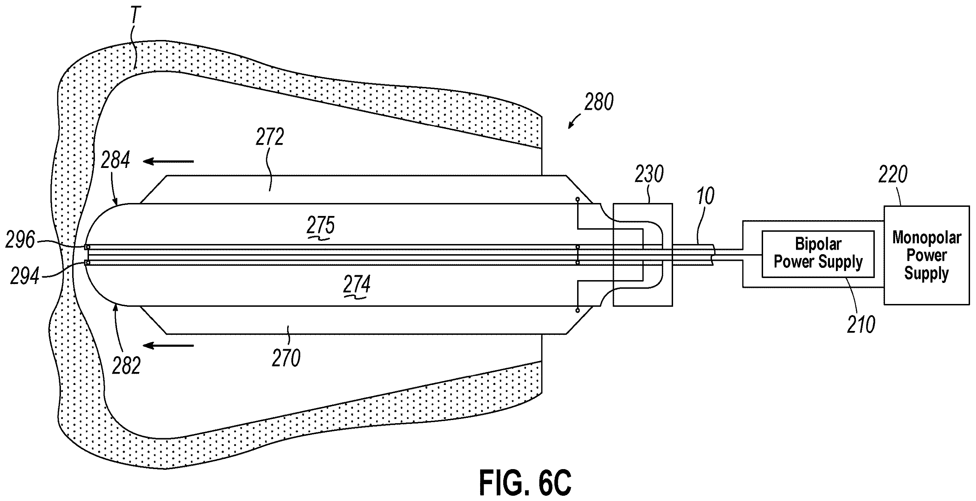

FIGS. 6A-6C depict end effector (280) being used to separate and ablate adjacent tissue to thereby gain access to a distal tissue portion. Either end effector (180, 280) may be used in the manor as shown. As shown in FIG. 6A, end effector (280) in the closed configuration may be inserted or wedged into an opening of tissue such that tissue compresses end effector (280). During insertion, operator may energize monopolar electrodes (270, 272) to ablate and cut tissue for easier insertion. As shown in FIG. 6B, once inserted to an end of tissue, operator may transition end effector (280) from the closed configuration to the open configuration to thus separate tissue further. During this transition, operator may energize monopolar electrodes (270, 272) to ablate and cut tissue for easier separation. Operator may continue to ablate and cut tissue while end effector (280) is in the open configuration to thereby ensure proper tissue separation. As shown in FIG. 6C, may then be transitioned from the open configuration to the closed configuration. Ablated and cut tissue may remain separated and out of the way for further distal advancement of end effector (280). The steps shown in FIGS. 6A-6C may be repeated indefinitely until a target tissue site has been reached. Target tissue site may then be evaluated, sealed, cut, and ablated using remaining portions of end effector (280) as described above.

### II. Illustrative Combinations

The following examples relate to various non-exhaustive ways in which the teachings herein may be combined or applied. The following examples are not intended to restrict the coverage of any claims that may be presented at any time in this application or in subsequent filings of this application. No disclaimer is intended. The following examples are being provided for nothing more than merely illustrative purposes. It is contemplated that the various teachings herein may be arranged and applied in numerous other ways. It is also contemplated that some variations may omit certain features referred to in the below examples. Therefore, none of the aspects or features referred to below should be deemed critical unless otherwise explicitly indicated as such at a later date by the inventors or by a successor in interest to the inventors. If any claims are presented in this application or in subsequent filings related to this application that include additional features beyond those referred to below, those additional features shall not be presumed to have been added for any reason relating to patentability.

### Example 1

A surgical instrument, comprising: a shaft assembly; and an end effector distally extending from the shaft assembly in a distal direction, wherein the end effector comprises: a first jaw having a first inner portion and an opposing, first outer portion, a second jaw having a second inner portion and an opposing, second outer portion and configured to selectively move relative to the first jaw from a closed configuration toward an open configuration wherein the first and second inner portions face toward each other in the closed configuration, a first bipolar electrode secured to the first inner portion of the first jaw or the second inner portion of the second jaw, a second bipolar electrode secured to the first inner portion of the first jaw or the second inner portion of the second jaw, and a first monopolar electrode secured to the first outer portion of the first jaw or the second outer portion of the second jaw.

### Example 2

The surgical instrument of Example 1, wherein the first bipolar electrode is secured to the first inner portion of the first jaw, wherein the second bipolar electrode is secured to the second inner portion of the second jaw, and wherein the first monopolar electrode is secured to the first outer portion of the first jaw.

### Example 3

The surgical instrument of Example 1, wherein the first monopolar electrode is secured to the first outer portion of the first jaw, wherein the first outer portion of the first jaw includes a first outer surface, wherein the first monopolar electrode distally extends along the first outer surface.

### Example 4

The surgical instrument of any one or more of Examples 1 through 3, wherein the first monopolar electrode defines a fin shape.

### Example 5

The surgical instrument of any one or more of Examples 1 through 4, wherein the first monopolar electrode includes an electrode height and an electrode width, wherein the electrode height upwardly extends from the first outer surface in an upward direction, wherein the electrode width laterally extends along the first outer surface in a lateral direction transverse to the upward and distal directions, and wherein the electrode height is greater than the electrode width.

### Example 6

The surgical instrument of any one or more of Examples 1 through 5, wherein the first monopolar electrode includes a bulbous tip at a distal end thereof.

### Example 7

The surgical instrument of any one or more of Examples 1 through 6, wherein the first jaw is pivotable relative to the shaft assembly.

### Example 8

The surgical instrument of any one or more of Examples 1 through 7, wherein the second jaw is pivotable relative to the shaft assembly.

### Example 9

The surgical instrument of any one or more of Examples 1 through 8, wherein the first monopolar electrode is fixedly secured to the first jaw.

### Example 10

The surgical instrument of any one or more of Examples 1 through 9, wherein the end effector is sized to fit within a trocar.

### Example 11

A surgical instrument, comprising: a shaft assembly; and an end effector distally extending from the shaft assembly in a distal direction, wherein the end effector comprises: a first jaw having a first inner surface and an opposing, first outer surface, and a second jaw having a second inner surface and an opposing, second outer surface and configured to selectively pivot relative to the first jaw from a closed configuration toward an open configuration, wherein the first and second inner surfaces face toward each other in the closed configuration, a first bipolar electrode secured to the first inner surface of the first jaw, a second bipolar electrode secured to the second inner surface of the second jaw, and a first monopolar electrode secured to the first outer surface of the first jaw and distally extends along the first outer surface, wherein the first monopolar electrode defines a fin shape.

### Example 12

The surgical instrument of Example 11, further comprising a power supply having a bipolar power supply and a monopolar power supply, wherein the bipolar power supply is configured to supply energy to the first bipolar electrode and to the second bipolar electrode, wherein the monopolar power supply is configured to supply energy to the first monopolar electrode.

### Example 13

The surgical instrument of Example 12, wherein the bipolar power supply and the monopolar power supply are electrically isolated from one another.

### Example 14

The surgical instrument of any one or more of Examples 11 through 13, wherein the power supply is configured to selectively operate the bipolar power supply or the monopolar power supply.

### Example 15

The surgical instrument of any one or more of Examples 11 through 14, wherein the end effector further includes a second monopolar electrode positioned along a second outer surface of the second jaw.

### Example 16

The surgical instrument of Example 15, wherein the second monopolar electrode is shaped the same as the first monopolar electrode.

### Example 17

The surgical instrument of any one or more of Examples 11 through 16, wherein the first jaw includes an elongate length, wherein the first monopolar electrode is in a shape of a fin that extends along the elongate length of the first jaw.

### Example 18

A method of applying at least one of a monopolar energy and a bipolar energy to a tissue with a surgical instrument, the surgical instrument including (a) a shaft assembly; and (b) an end effector distally extending from the shaft assembly in a distal direction, wherein the end effector comprises: (i) a first jaw having a first inner portion and an opposing, first outer portion, and (ii) a second jaw having a second inner portion and an opposing, second outer portion and configured to selectively move relative to the first jaw from a closed configuration toward an open configuration wherein the first and second inner portions face toward each other in the closed configuration, (iii) a first bipolar electrode secured to the first inner portion of the first jaw or the second inner portion of the second jaw, (iv) a second bipolar electrode secured to the first inner portion of the first jaw or the second inner portion of the second jaw, and (v) a first monopolar electrode secured to the first outer portion of the first jaw or the second outer portion of the second jaw, the method comprising: selectively energizing the first monopolar electrode or the first and second bipolar electrodes thereby applying at least one of the monopolar energy and the bipolar energy to the tissue.

### Example 19

The method of Example 18, wherein selectively energizing further includes: selectively energizing the first monopolar electrode thereby applying the monopolar energy to the tissue; and selectively energizing the first and second bipolar electrodes thereby applying the monopolar energy to the tissue.

### Example 20

The method of any one or more of Examples 18 through 19, further comprising electrically isolating the monopolar electrode from each of the first and second bipolar electrodes.

### III. Miscellaneous

It should be understood that any of the versions of the instruments described herein may include various other features in addition to or in lieu of those described above. By way of example only, any of the devices herein may also include one or more of the various features disclosed in any of the various references that are incorporated by reference herein. Various suitable ways in which such teachings may be combined will be apparent to those of ordinary skill in the art.

While the examples herein are described mainly in the context of electrosurgical instruments, it should be understood that various teachings herein may be readily applied to a variety of other types of devices. By way of example only, the various teachings herein may be readily applied to other types of electrosurgical instruments, tissue graspers, tissue retrieval pouch deploying instruments, surgical staplers, surgical clip appliers, ultrasonic surgical instruments, etc. It should also be understood that the teachings herein may be readily applied to any of the instruments described in any of the references cited herein, such that the teachings herein may be readily combined with the teachings of any of the references cited herein in numerous ways. Other types of instruments into which the teachings herein may be incorporated will be apparent to those of ordinary skill in the art.

It should be understood that any one or more of the teachings, expressions, embodiments, examples, etc. described herein may be combined with any one or more of the other teachings, expressions, embodiments, examples, etc. that are described herein. The above-described teachings, expressions, embodiments, examples, etc. should therefore not be viewed in isolation relative to each other. Various suitable ways in which the teachings herein may be combined will be readily apparent to those of ordinary skill in the art in view of the teachings herein. Such modifications and variations are intended to be included within the scope of the claims.

It should be appreciated that any patent, publication, or other disclosure material, in whole or in part, that is said to be incorporated by reference herein is incorporated herein only to the extent that the incorporated material does not conflict with existing definitions or other disclosure material set forth in this disclosure. As such, and to the extent necessary, the disclosure as explicitly set forth herein supersedes any conflicting material incorporated herein by reference. Any material, or portion thereof, that is said to be incorporated by reference herein, but which conflicts with existing definitions or other disclosure material set forth herein will only be incorporated to the extent that no conflict arises between that incorporated material and the existing disclosure material.

Versions of the devices described above may have application in conventional medical treatments and procedures conducted by a medical professional, as well as application in robotic-assisted medical treatments and procedures. By way of example only, various teachings herein may be readily incorporated into a robotic surgical system such as the DAVINCI^{™} system by Intuitive Surgical, Inc., of Sunnyvale, California. Similarly, those of ordinary skill in the art will recognize that various teachings herein may be readily combined with various teachings of U.S. Pat. No. 6,783,524, entitled "Robotic Surgical Tool with Ultrasound Cauterizing and Cutting Instrument," published August 31, 2004, the disclosure of which is incorporated by reference herein, in its entirety.

Versions described above may be designed to be disposed of after a single use, or they can be designed to be used multiple times. Versions may, in either or both cases, be reconditioned for reuse after at least one use. Reconditioning may include any combination of the steps of disassembly of the device, followed by cleaning or replacement of particular pieces, and subsequent reassembly. In particular, some versions of the device may be disassembled, and any number of the particular pieces or parts of the device may be selectively replaced or removed in any combination. Upon cleaning and/or replacement of particular parts, some versions of the device may be reassembled for subsequent use either at a reconditioning facility, or by an operator immediately prior to a procedure. Those skilled in the art will appreciate that reconditioning of a device may utilize a variety of techniques for disassembly, cleaning/replacement, and reassembly. Use of such techniques, and the resulting reconditioned device, are all within the scope of the present application.

By way of example only, versions described herein may be sterilized before and/or after a procedure. In one sterilization technique, the device is placed in a closed and sealed container, such as a plastic or TYVEK bag. The container and device may then be placed in a field of radiation that can penetrate the container, such as gamma radiation, x-rays, or high-energy electrons. The radiation may kill bacteria on the device and in the container. The sterilized device may then be stored in the sterile container for later use. A device may also be sterilized using any other technique known in the art, including but not limited to beta or gamma radiation, ethylene oxide, or steam.

Having shown and described various embodiments of the present invention, further adaptations of the methods and systems described herein may be accomplished by appropriate modifications by one of ordinary skill in the art without departing from the scope of the present invention. Several of such potential modifications have been mentioned, and others will be apparent to those skilled in the art. For instance, the examples, embodiments, geometrics, materials, dimensions, ratios, steps, and the like discussed above are illustrative and are not required. Accordingly, the scope of the present invention should be considered in terms of the following claims and is understood not to be limited to the details of structure and operation shown and described in the specification and drawings.

## Claims

1. A surgical instrument, comprising:
(a) a shaft assembly; and
(b) an end effector distally extending from the shaft assembly in a distal direction, wherein the end effector comprises:
(i) a first jaw having a first inner portion and an opposing, first outer portion,
(ii) a second jaw having a second inner portion and an opposing, second outer portion and configured to selectively move relative to the first jaw from a closed configuration toward an open configuration wherein the first and second inner portions face toward each other in the closed configuration,
(iii) a first bipolar electrode secured to the first inner portion of the first jaw or the second inner portion of the second jaw,
(iv) a second bipolar electrode secured to the first inner portion of the first jaw or the second inner portion of the second jaw, and
(v) a first monopolar electrode secured to the first outer portion of the first jaw or the second outer portion of the second jaw.

2. The surgical instrument of claim 1, wherein the first bipolar electrode is secured to the first inner portion of the first jaw, wherein the second bipolar electrode is secured to the second inner portion of the second jaw, and wherein the first monopolar electrode is secured to the first outer portion of the first jaw.

3. The surgical instrument of claim 1 or claim 2, wherein the first monopolar electrode is secured to the first outer portion of the first jaw, wherein the first outer portion of the first jaw includes a first outer surface, wherein the first monopolar electrode distally extends along the first outer surface.

4. The surgical instrument of any preceding claim, wherein the first monopolar electrode defines a fin shape.

5. The surgical instrument of claim 3, or claim 4 when dependent on claim 3, wherein the first monopolar electrode includes an electrode height and an electrode width, wherein the electrode height upwardly extends from the first outer surface in an upward direction, wherein the electrode width laterally extends along the first outer surface in a lateral direction transverse to the upward and distal directions, and wherein the electrode height is greater than the electrode width.

6. The surgical instrument of any preceding claim, wherein the first monopolar electrode includes a bulbous tip at a distal end thereof.

7. The surgical instrument of claim 1, wherein one or both of the first jaw and the second jaw are pivotable relative to the shaft assembly.

8. The surgical instrument of any preceding claim, wherein the first monopolar electrode is fixedly secured to the first jaw.

9. The surgical instrument of any preceding claim, wherein the end effector is sized to fit within a trocar.

10. A surgical instrument, comprising:
(a) a shaft assembly; and
(b) an end effector distally extending from the shaft assembly in a distal direction, wherein the end effector comprises:
(i) a first jaw having a first inner surface and an opposing, first outer surface, and
(ii) a second jaw having a second inner surface and an opposing, second outer surface and configured to selectively pivot relative to the first jaw from a closed configuration toward an open configuration, wherein the first and second inner surfaces face toward each other in the closed configuration,
(iii) a first bipolar electrode secured to the first inner surface of the first jaw,
(iv) a second bipolar electrode secured to the second inner surface of the second jaw, and
(v) a first monopolar electrode secured to the first outer surface of the first jaw and distally extends along the first outer surface, wherein the first monopolar electrode defines a fin shape.

11. The surgical instrument of claim 10, further comprising a power supply having a bipolar power supply and a monopolar power supply, wherein the bipolar power supply is configured to supply energy to the first bipolar electrode and to the second bipolar electrode, wherein the monopolar power supply is configured to supply energy to the first monopolar electrode.

12. The surgical instrument of claim 11, wherein the bipolar power supply and the monopolar power supply are electrically isolated from one another.

13. The surgical instrument of claim 11 or claim 12, wherein the power supply is configured to selectively operate the bipolar power supply or the monopolar power supply.

14. The surgical instrument of claim any one of claims 10 to 13, wherein the end effector further includes a second monopolar electrode positioned along a second outer surface of the second jaw, preferably wherein the second monopolar electrode is shaped the same as the first monopolar electrode.

15. The surgical instrument of any one of claims 10 to 14, wherein the first jaw includes an elongate length, wherein the first monopolar electrode is in a shape of a fin that extends along the elongate length of the first jaw.
